(19)

Europäisches Patentamt
European Patent Office
Office européen des brevets

(11) **EP 3 701 861 A1**

(12) **EUROPEAN PATENT APPLICATION**
published in accordance with Art. 153(4) EPC

(43) Date of publication:
**02.09.2020 Bulletin 2020/36**

(51) Int Cl.:
**A61B 5/0275** (2006.01)     **A61B 10/00** (2006.01)

(21) Application number: **18870820.0**

(22) Date of filing: **24.10.2018**

(86) International application number:
**PCT/JP2018/039524**

(87) International publication number:
**WO 2019/082938 (02.05.2019 Gazette 2019/18)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **25.10.2017   JP 2017206365**

(71) Applicants:
• **National Cerebral and Cardiovascular Center**
  **Suita-shi, Osaka 564-8565 (JP)**
• **Hamamatsu Photonics K.K.**
  **Hamamatsu-shi, Shizuoka 435-8558 (JP)**

(72) Inventors:
• **YOSHITANI Kenji**
  **Suita-shi**
  **Osaka 565-8565 (JP)**
• **KATO Shinya**
  **Suita-shi**
  **Osaka 565-8565 (JP)**
• **EZAKA Mariko**
  **Suita-shi**
  **Osaka 565-8565 (JP)**
• **OZAKI Takeo**
  **Hamamatsu-shi**
  **Shizuoka 435-8558 (JP)**

(74) Representative: **Hoffmann Eitle**
**Patent- und Rechtsanwälte PartmbB**
**Arabellastraße 30**
**81925 München (DE)**

(54) **CEREBRAL BLOOD FLOW MEASUREMENT METHOD AND MEASUREMENT DEVICE**

(57)     Provided is a cerebral blood flow measurement method of quantitatively measuring cerebral blood flow, the measurement method including: a tracer introducing step of introducing a tracer into a blood vessel; a light applying step of applying measurement light including an absorption wavelength of the tracer to a head; a light detecting step of detecting the measurement light propagating in the head and generating a detection signal based on light intensity of the measurement light; and a calculation step of calculating cerebral blood flow on the basis of a relative change over time $\Delta Q$ of a concentration of the tracer in a brain tissue which is acquired on the basis of the detection signal and a predetermined relationship between a change over time of a concentration $Pa$ of the tracer in an artery of the head and cerebral blood flow. The calculation step includes calculating the concentration $Pa$ using the amplitude of a pulse-wave component of the relative change $\Delta Q$.

*Fig.1*

EP 3 701 861 A1

**Description**

**Technical Field**

An aspect of the invention relates to a cerebral blood flow measurement method and a cerebral blood flow measurement device.

**Background Art**

Measurement of cerebral blood flow is very useful for diagnosis of brain diseases such as moyamoya disease and a subarachnoidal hemorrhage. Accordingly, in clinical spots, there is demand for simple and accurate measurement of cerebral blood flow of a patient with a brain disease. In the related art, measurement of cerebral blood flow is performed using positron emission tomography (PET) or single photon emission tomography (SPECT) by injecting a radioactive isotope into veins. However, with this method, it is necessary to carry a patient to equipment of a department of radiology and it is difficult to measure cerebral blood flow of a severely ill patient.

Therefore, use of a near-infrared spectroscopy (NIRS) method is conceivable. That is, a tracer having an absorption wavelength in a near-infrared band (for example, indocyanine green (ICG)) is injected into arteries or veins, near-infrared light is applied to the head, and near-infrared light propagating in the head is detected (for example, see Non Patent Literatures 1 to 6). Since a rate of increase of a concentration change of a tracer is proportional to a cerebral blood flow rate, cerebral blood flow can be measured simply, for example, using a small device such as an NIRS device that measures a blood hemoglobin concentration or an oxygen saturation according to this method. Accordingly, without carrying a patient, a device may be placed in the vicinity of a bed and perform measurement in a patient room or at the time of operation, catheter inspection, or the like.

**Citation List**

**Non Patent Literature**

Non Patent Literature 1: H. W. Schytz et al., "Changes in cerebral blood flow after acetazolamide: an experimental study comparing near-infrared spectroscopy and SPECT," Eur J Neurol. Author manuscript; available in PMC 2009 November 25
Non Patent Literature 2: Bendicht P. Wagner et al., "Reproducibility of the blood flow index as noninvasive, bedside estimation of cerebral blood flow," Intensive Care Med29, pp. 196-200, (2003)
Non Patent Literature 3: Christoph Terborg et al., "Noninvasive Assessment of Cerebral Perfusion and Oxygenation in Acute Ischemic Stroke by Near-Infrared Spectroscopy," European Neurology, Vol. 62, pp. 338-343, (2009)
Non Patent Literature 4: Felix Gora et al., "Noninvasive Measurement of Cerebral Blood Flow in Adults Using Near-Infrared Spectroscopy and Indocyanine Green: A Pilot Study," Journal of Neurosurgical Anesthesiology Vol. 14, No. 3, pp. 218-222
Non Patent Literature 5: Kuebler WM, Sckell A and Habler O. et al., "Noninvasive measurement of regional cerebral blood flow by near-infrared spectroscopy and indocyanine green," J Cereb Blood Metab, Vol. 18, pp. 445-456 (1998)
Non Patent Literature 6: Kato S, Yoshitani K and Ohnishi Y, "Cerebral Blood Flow Measurement by Near-Infrared Spectroscopy During Carotid Endarterectomy," Journal of Neurosurgical Anesthesiology, Vol. 28(4), pp. 291-295 (2016)

**Summary of Invention**

**Technical Problem**

However, the NIRS method is a method of observing a quantitative change of a light-absorbing material by measuring a relative change of a concentration of the light-absorbing material and is not a method of measuring an absolute change (a quantitative value) of the light-absorbing material. Accordingly, when cerebral blood flow is measured using NIRS as described above, trends in changes of cerebral blood flow can be understood but there is a problem in that it is difficult to quantitatively measure cerebral blood flow.

An aspect of the invention is made in consideration of the above-mentioned circumstances and an objective thereof is to provide a cerebral blood flow measurement method and a cerebral blood flow measurement device that can quantitatively measure cerebral blood flow using a simple device.

**Solution to Problem**

A cerebral blood flow measurement method according to an aspect of the invention is a cerebral blood flow measurement method of quantitatively measuring cerebral blood flow, the measurement method including: a tracer introducing step of introducing a tracer into a blood vessel; a light applying step of applying measurement light including an absorption wavelength of the tracer to a head; a light detecting step of detecting the measurement light propagating in the head and generating a detection signal based on light intensity of the measurement light; and a calculation step of calculating cerebral blood flow on the basis of a relative change over time $\Delta Q$ of a concentration of the tracer in a brain tissue which is acquired on the basis of the detection signal and a predetermined relationship between a change over time of a concentration Pa of the tracer in an artery of the head and cerebral blood flow, wherein the calculation step includes calculating the change over time of the concentration Pa using a change over time of the amplitude of a pulse-wave component of the relative change $\Delta Q$.

A cerebral blood flow measurement device according to an aspect of the invention is a cerebral blood flow measurement device that quantitatively measures cerebral blood flow, the measurement device including: a light applying unit configured to apply measurement light including an absorption wavelength of a tracer which is introduced into a blood vessel to a head; a light detecting unit configured to detect the measurement light propagating in the head and generates a detection signal based on light intensity of the measurement light; and a calculation unit configured to calculate cerebral blood flow on the basis of a relative change over time $\Delta Q$ of a concentration of the tracer in a brain tissue which is acquired on the basis of the detection signal and a predetermined relationship between a change over time of a concentration Pa of the tracer in an artery of the head and cerebral blood flow, wherein the calculation unit calculates the change over time of the concentration Pa using a change over time of the amplitude of a pulse-wave component of the relative change $\Delta Q$.

As described above, when a tracer is introduced into a blood vessel, the tracer concentration in a brain tissue increases gradually. A rate of increase thereof is proportional to a cerebral blood flow rate. In the measurement method and the measurement device, measurement light including an absorption wavelength of the tracer is applied to the head and the measurement light propagating in the head is detected. At this time, since the measurement light is absorbed according to the tracer concentration in the brain tissue, the relative change $\Delta Q$ can be calculated on the basis of a predetermined relationship between the amount of absorbed light and the relative change $\Delta Q$ over time of the tracer concentration.

The relative change $\Delta Q$ over time of the tracer concentration in the brain tissue, the change over time of the concentration Pa of the tracer in the artery of the head, and the cerebral blood flow satisfy a relationship which is expressed by Equation (1) which will be described later (Fick's principle). The inventor found that there is a close relationship between the change over time of the amplitude of the pulse-wave component of the relative change $\Delta Q$ and the concentration Pa of the tracer in the artery of the head. In the measurement method and the measurement device, since the change over time of the tracer concentration Pa is calculated using the change over time of the amplitude of the pulse-wave component of the relative change $\Delta Q$, it is possible to calculate the cerebral blood flow. Accordingly, with the measurement method and the measurement device, it is possible to quantitatively measure cerebral blood flow using a simple device. Since the change over time of the concentration Pa in a measurement target region can be directly measured, it is possible to improve measurement accuracy.

In the measurement method and the measurement device, the calculation step may include (the calculation unit may perform) extracting the pulse-wave component by performing a filtering process of removing a frequency component lower than a predetermined frequency on the detection signal. Alternatively, the calculation step may include (the calculation unit may perform) extracting the pulse-wave component by performing a filtering process of removing a frequency component lower than a predetermined frequency on the relative change $\Delta Q$. With this method (device), it is possible to easily and accurately extract the pulse-wave component of the relative change $\Delta Q$ and to acquire the amplitude of the pulse-wave component. In this case, the predetermined frequency may be equal to or greater than 10 Hz and equal to or less than 100 Hz. According to the inventor's knowledge, it is possible to accurately extract the pulse-wave component by removing a frequency component less than such a frequency.

In the measurement method and the measurement device, a sampling frequency of the relative change $\Delta Q$ may be greater than 10 Hz. In general, a heart rate of a person in a stabilized state ranges from about 60 per minute to 75 per minute (that is, about 1 Hz to 1.25 Hz). Accordingly, for example, by measuring the relative change $\Delta Q$ at a sampling frequency sufficiently greater (a sampling period shorter) than the heart rate, it is possible to appropriately acquire the amplitude of the pulse-wave component of the relative change $\Delta Q$.

In the measurement method and the measurement device, the calculation step may include (the calculation unit may perform) calculating a change over time of the tracer concentration Pa on the basis of the change over time of the amplitude of the pulse-wave component and an absolute concentration of the tracer in an artery of the head at a certain time. Accordingly, it is possible to appropriately calculate the change over time of the tracer concentration Pa. In this case, the absolute concentration of the tracer in an artery of the head may be measured using a dye dilution method. Similarly, the measurement device may further include a measurement unit that measures the absolute concentration of the tracer in an artery of the head using a dye dilution method.

The measurement method may further include a hemoglobin measuring step of measuring an absolute hemoglobin concentration in an artery in advance before the tracer introducing step, the light applying step may include applying the measurement light including an absorption wavelength of hemoglobin to the head, and the calculation step may include calculating a change over time of a relative hemoglobin concentration in a brain tissue on the basis of the detection signal and calculating the change over time of the concentration Pa on the basis of the absolute hemoglobin concentration, the change over time of the amplitude of the pulse-wave component of the change over time of the relative hemoglobin concentration, and the change over time of the amplitude of the pulse-wave component of the relative change $\Delta Q$. Similarly, the measurement device may further include a measurement unit configured to measure an absolute hemoglobin concentration in an artery in advance before introducing the tracer, the light applying unit may apply the measurement light including an absorption wavelength of hemoglobin to the head, and the calculation unit may calculate a change over time of a relative hemoglobin concentration in a brain tissue on the basis of the detection signal and calculate the change over time of the concentration Pa on the basis of the absolute hemoglobin concentration, the change over time of the amplitude of the pulse-wave component of the change over time of the relative hemoglobin concentration, and the change over time of the amplitude of the pulse-wave component of the relative change $\Delta Q$. For example, with this device and method, it is possible to appropriately calculate the change over time of the tracer concentration Pa. Since the measurement unit that measures the absolute concentration of the tracer using the dye dilution method is not necessary, it is possible to simplify a device configuration.

In the measurement method, indocyanine green may be used as the tracer. Similarly, in the measurement device, indocyanine green may be used as the tracer. Indocyanine green has an absorption wavelength in a near-infrared band, is used for various types of inspection in the related art, and is safe and inexpensive. Accordingly, by using indocyanine green as the tracer, it is possible to safely and inexpensively measure cerebral blood flow.

In the measurement method and the measurement device, the absorption wavelength of the tracer may be included in a near-infrared band. Since light in the near-infrared band can easily pass through various tissues of the head, it is possible to accurately measure a tracer concentration using a tracer of which an absorption wavelength is included in the near-infrared band.

## Advantageous Effects of Invention

With the cerebral blood flow measurement method and the cerebral blood flow measurement device according to the aspects of the invention, it is possible to quantitatively measure cerebral blood flow using a simple device.

## Brief Description of Drawings

FIG. 1 is a diagram schematically illustrating a configuration of a measurement device according to an embodiment.

FIG. 2 is a graph illustrating an example of a change over time of a relative change $\Delta Q$ of an ICG concentration.

FIG. 3 is a diagram illustrating filter characteristics of a digital filter.

FIG. 4 is a graph illustrating results of removing frequency components less than a predetermined frequency from frequency components included in a relative change $\Delta Q$ over time of an ICG concentration using the digital filter illustrated in FIG. 3 and extracting a pulse-wave component based on a heartbeat.

FIGS. 5(a) and 5(b) are diagrams illustrating the concept of a filtering process.

FIG. 6 is a flowchart illustrating constituent steps of a method of operating the measurement device (a cerebral blood flow measurement method).

FIG. 7(a) is a graph illustrating cerebral blood flow of a plurality of sample subjects who were measured using PET and FIG. 7(b) is a graph illustrating cerebral blood flow which was measured from the same sample subjects at the same time as measured using PET using the measurement device and the measurement method according to this embodiment.

FIG. 8 is a diagram schematically illustrating a configuration of a measurement device according to a modified example.

FIG. 9(a) is a graph conceptually illustrating a change over time of a pulse-wave component which is extracted from a relative change $\Delta O_2Hb$ and FIG. 9(b) is a graph conceptually illustrating a change over time of a pulse-wave component which is extracted from a relative change $\Delta Q$.

FIG. 10 is a flowchart illustrating a method of operating a measurement device (a cerebral blood flow measurement method) according to a modified example.

**Description of Embodiments**

Hereinafter, embodiments of a cerebral blood flow measurement method and a cerebral blood flow measurement device will be described in detail with reference to the accompanying drawings. In description with reference to the drawings, the same elements will be referred to by the same reference signs and description thereof will not be repeated.

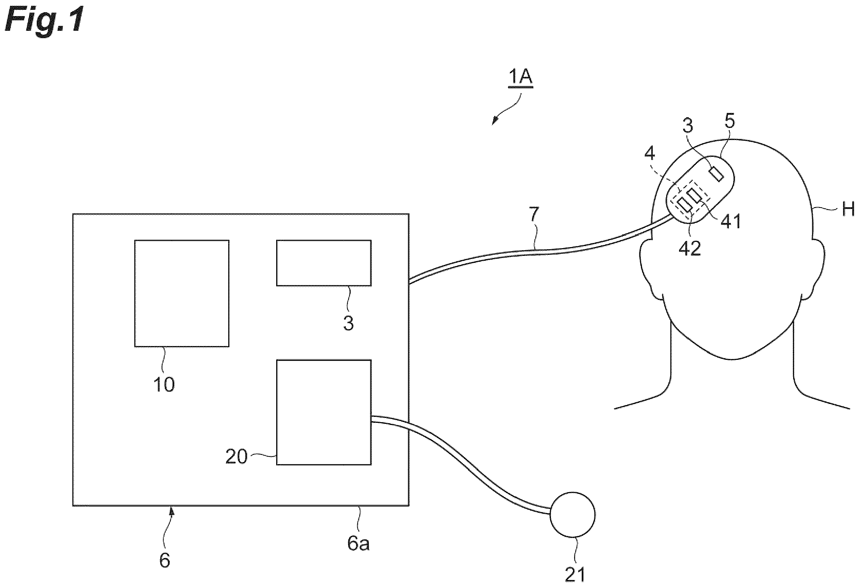

FIG. 1 is a diagram schematically illustrating a configuration of a measurement device 1A according to an embodiment. The measurement device 1A according to this embodiment is a device that quantitatively measures cerebral blood flow (CBF) and includes a light source (a light applying unit) 3, a light detector (a light detecting unit) 4, a calculation unit 10, and a measurement unit 20 as illustrated in FIG. 1. The light source 3 and the light detector 4 are attached to a probe 5, and the calculation unit 10 and the measurement unit 20 are accommodated in a casing 6a of a main body 6. The probe 5 is attached to a measurement target region of a head H of a sample subject. In the drawing, both the calculation unit 10 and the measurement unit 20 are accommodated in the common casing 6a, but the calculation unit 10 and the measurement unit 20 may be accommodated in individual casings. The light applying unit may have a configuration in which the light source 3 is accommodated in the casing 6a of the main body 6 and measurement light L1 output from the light source 3 is applied to the head H via an optical fiber connecting the main body 6 and the probe 5. The light detecting unit may have a configuration in which the light detector 4 is accommodated in the casing 6a of the main body 6 and measurement light L1 propagating in the head H is detected by the light detector 4 via the optical fiber connecting the main body 6 and the probe 5.

The light source 3 includes, for example, a light emitting element such as a light emitting diode (LED) or a laser diode (LD) and a circuit that drives the light emitting element. The light source 3 outputs measurement light L1 which is applied to the head H. The light source 3 and the main body 6 are connected to each other via a cable 7. The measurement light L1 is light of a plurality of wavelengths (for example, three wavelengths) including an absorption wavelength of a tracer which is introduced into a blood vessel. In the following description, the wavelengths of the measurement light L1 are defined as $\lambda_1$, $\lambda_2$, and $\lambda_3$ (where $\lambda_1$, $\lambda_2$, and $\lambda_3$ are different wavelengths). A tracer is a dye such as indocyanine green (ICG) and is injected into arteries or veins outside the head of a sample subject immediately before measurement using the measurement device 1A. Since the ICG is coupled to a plasma protein in blood after being injected into a blood vessel, the ICG circulates in the blood vessel and does not move out of the blood vessel. An absorption wavelength of the ICG is 805 nm in a state in which it is coupled to the plasma protein, and is included in a near-infrared band. Accordingly, the wavelengths of the measurement light L1 are around 805 nm and are included in the near-infrared band. Specifically, the wavelengths of the measurement light L1 are included in a range of 700 nm to 850 nm. An output wavelength and an emission time of the light source 3 are controlled by a calculation unit 10 which will be described later. The light source 3 may be connected to the main body 6 by wireless communication.

A light detector 4 detects measurement light L1 propagating in the head H and generates an electrical detection signal S1 corresponding to light intensity of the measurement light L1. The light detector 4 is disposed with a gap from the light source 3 in the probe 5. The light detector 4 includes, for example, a light detecting element 41 having sensitivity in a wavelength band including the wavelengths of the measurement light L1 and has sensitivity, for example, in a near-infrared band. The light detecting element 41 is, for example, a photodiode such as an avalanche photodiode or a silicon photodiode. The light detector 4 provides the generated detection signal S1 to the calculation unit 10 via the cable 7 connecting the probe 5 and the main body 6. The light detector 4 may further include a preamplifier 42 that integrates and amplifies a photoelectric current which is output from the light detecting element 41. Accordingly, the light detector 4 can detect a weak signal with high sensitivity, generate a detection signal S1, and transmit the detection signal S1 to the main body 6 via the cable 7. The light detecting element 41 may be a one-dimensional detector or a two-dimensional detector. The light detector 4 may be connected to the main body 6 by wireless communication.

The calculation unit 10 calculates cerebral blood flow on the basis of a relative change over time of a tracer concentration (a change over time from an initial value (a tracer concentration at time 0)) in a brain tissue which is acquired on the basis of the detection signal S1 and a predetermined relationship between the tracer concentration in arteries of the head H and the cerebral blood flow. The calculation unit 10 is realized, for example, by a computer. The computer includes a CPU and a memory and operates by executing a program. Examples of the computer include a personal computer, a microcomputer, a cloud server, and a smart device (such as a smartphone or a tablet terminal).

A measurement unit 20 measures an absolute concentration of the tracer in arteries of the head H using a dye dilution method. The measurement unit 20 is suitably realized, for example, by a dye densito-gram (DDG) analyzer. Examples of the DDG analyzer include the DDG-3000 series made by Nihon Kohden. Measurement methods described in the following documents can be employed for the measurement unit 20

- Takehiko Iijima et al., "Cardiac Output And Circulating Blood Volume Analysis By Pulse Dye-Densitometry," journal of Clinical Monitoring, Vol. 13, No. 2, pp. 81-89, (1997)
- Takasuke Imai et al., "Measurement of Cardiac Output by Pulse Dye Densitometry Using Indocyanine Green," Anesthesiology, Vol. 87, No. 4, (1997)

- Masaki Haruna et al., "Blood Volume Measurement at the Bedside UsingICG Pulse Spectrophotometry," Anesthesiology, Vol. 89, No. 6, (1998)
- Takehiko Iijima et al., "Circulating Blood Volume Measured by Pulse Dye-Densitometry," Anesthesiology, Vol. 89, No. 6, (1998)
- Takasuke Imai et al., "Measurement of Blood Concentration of Indogyanine Green by Pulse Dye Densitometry Comparison with the Conventional Spectrophotometric Method," Journal of Clinical Monitoring and Computing, Vol. 14, pp. 477-484, (1998)

A probe 21 for measurement extends from the measurement unit 20, and the probe 21 is attached to a part of a body of a sample subject (for example, a finger or a nose). Measurement of an absolute concentration of the tracer by the measurement unit 20 is performed at least once with every measurement of cerebral blood flow.

Details of the cerebral blood flow calculating method which is performed by the calculation unit 10 will be described below. In the following description, it is assumed that ICG is used as the tracer. When Q ($\mu$mol/100 g) denotes a tracer concentration in a brain tissue, Pa ($\mu$mol/ml) denotes an absolute tracer concentration in arteries of the head H, Pv ($\mu$mol/ml) denotes an absolute tracer concentration in veins of the head H, and F (ml/(100 g·min)) denotes cerebral blood flow, these satisfy the following relationship (Fick's principle).

[Math. 1]

$$\frac{dQ}{dt} = F \cdot (Pa - Pv) \quad \cdots \quad (1)$$

Within a time in which a change of the tracer concentration does not appear in the veins of the head H (about 10 seconds) after a tracer has been injected into a region other than the head H, Pv=0 can be considered and thus the above-mentioned expression becomes the following expression.

[Math. 2]

$$\frac{dQ}{dt} = F \cdot Pa \quad \cdots \quad (2)$$

By integrating both sides, the following expression is established.

[Math. 3]

$$\Delta Q = F \cdot \int_0^t Pa \cdot dt \quad \cdots \quad (3)$$

Accordingly, the cerebral blood flow F is calculated as follows.

[Math. 4]

$$F = \frac{\Delta Q}{\int_0^t Pa \cdot dt} \quad \cdots \quad (4)$$

The relative change $\Delta Q$ of the tracer concentration in a brain tissue is a change of the tracer concentration from time 0 to time t, and is calculated as follows by the calculation unit 10. Values of the detection signal S1 corresponding to detected light wavelengths $\lambda_1$, $\lambda_2$, and $\lambda_3$ at time $T_0$ are defined as $D_{\lambda 1}(T_0)$ to $D_{\lambda 3}(T_0)$ and the values at time $T_1$ are defined as $D_{\lambda 1}(T_1)$ to $D_{\lambda 3}(T_1)$, changes of detected light intensities at times $T_0$ to $T_1$ are expressed by Expressions (5) to (7).

[Math. 5]

$$\Delta OD_1(T_1) = \log\left(\frac{D_{\lambda 1}(T_1)}{D_{\lambda 1}(T_0)}\right) \quad \cdots \quad (5)$$

[Math. 6]

$$\Delta OD_2(T_1) = \log\left(\frac{D_{\lambda 2}(T_1)}{D_{\lambda 2}(T_0)}\right) \quad \cdot \cdot \cdot \quad (6)$$

[Math. 7]

$$\Delta OD_3(T_1) = \log\left(\frac{D_{\lambda 3}(T_1)}{D_{\lambda 3}(T_0)}\right) \quad \cdot \cdot \cdot \quad (7)$$

In Expressions (5) to (7), $\Delta OD1(T_1)$ denotes a change over time of the detected light intensity of a wavelength $\lambda_1$, $\Delta OD_2(T_1)$ denotes a change over time of the detected light intensity of a wavelength $\lambda_2$, and $\Delta OD_{3(}T_1)$ denotes a change over time of the detected light intensity of a wavelength $\lambda_3$.

When relative changes over time of the concentrations of oxygenated hemoglobin, deoxygenated hemoglobin, and ICG from time $T_0$ to time $_{Tl}$ are defined as $\Delta O_2Hb(T_1)$, $\Delta HHb(T_1)$, and $\Delta ICG(T_1)$, respectively, these can be calculated by Expression (8).

[Math. 8]

$$\begin{pmatrix}\Delta O_2\text{Hb}(T_1) \\ \Delta HHb(T_1) \\ \Delta ICG(T_1)\end{pmatrix} = \begin{pmatrix}b_{11} & b_{12} & b_{13} \\ b_{21} & b_{22} & b_{23} \\ b_{31} & b_{32} & b_{33}\end{pmatrix}\begin{pmatrix}\Delta OD_1(T_1) \\ \Delta OD_2(T_1) \\ \Delta OD_3(T_1)\end{pmatrix} \quad \cdot \cdot \cdot \quad (8)$$

In Expression (8), coefficients $b_{11}$ to $b_{33}$ are constants calculated from extinction coefficients of $O_2Hb$, HHb, and ICG for light of the wavelengths $\lambda_1$, $\lambda_2$, and $\lambda_3$. The reason of addition of $\Delta O_2Hb(T_1)$ and $\Delta HHb(T_1)$ to Expression (8) is that the absorption wavelength of ICG and the absorption wavelengths of oxygenated hemoglobin and deoxygenated hemoglobin are very close to each other and an influence of such absorption is considered. The calculation unit 10 repeatedly perform the calculation on the detection signal S1 from the light detecting element 41 and periodically calculates a relative change over time $\Delta Q$ ($=\Delta ICG(T_1)$) of the ICG concentration. The calculation period at this time is sufficiently shorter than a cardiac cycle of a person, and the calculation period is converted to a sampling frequency which is greater than 10 Hz and is, for example, 20 Hz.

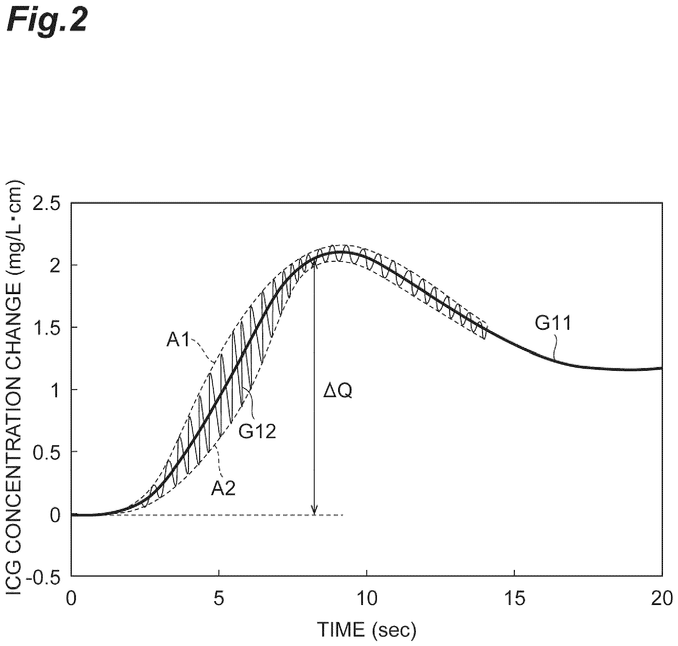

FIG. 2 is a graph illustrating an example of a change over time of the relative change $\Delta Q$ of the ICG concentration. In FIG. 2, the vertical axis represents the relative change $\Delta Q$ (unit: mg/l·cm) and the horizontal axis represents time (unit: second, a time point of injection of ICG is defined as 0 seconds). In FIG. 2, graph G11 indicates a principal change of the relative change $\Delta Q$ and graph G12 indicates a change of pulse-wave component of the relative change $\Delta Q$. With reference to graph G11, the relative change $\Delta Q$ of the ICG concentration is zero for a short time after injection of ICG and the relative change $\Delta Q$ increases gradually and reaches a peak at a certain time. Thereafter, the relative change $\Delta Q$ decreases gradually and is settled at a certain magnitude. On the other hand, as indicated by graph G12, the relative change $\Delta Q$ of the ICG concentration includes a pulse-wave component due to a heartbeat. The period of the pulse-wave component is the same as the cardiac cycle. The amplitude of the pulse-wave component reaches a peak during an increase of the relative change $\Delta Q$ and the amplitude of the pulse-wave component decreases when the relative change $\Delta Q$ reaches the peak. In the drawing, a dotted line A1 is a line connecting upper limits of the amplitude of the pulse-wave component, and a dotted line A2 is a line connecting lower limits of the amplitude of the pulse-wave component. The amplitude of the pulse-wave component at a certain time has the same meaning as an interval between the dotted line A1 and the dotted line A2 at that time.

In this embodiment, the pulse-wave component is extracted and a change over time of the amplitude thereof is measured in order to acquire a tracer concentration Pa in arteries of the head H. Specifically, the calculation unit 10 performs a filtering process of removing frequency components less than a predetermined frequency on the relative change $\Delta Q$ of the ICG concentration or the detection signal S1. In consideration of a heartbeat frequency (1 Hz to 1.25 Hz) in a stabilized state, the predetermined frequency (the sampling frequency) may be, for example, equal to or greater than 10 Hz and equal to or less than 100 Hz. An example of the filtering process which is performed by the calculation unit 10 will be described below. In the following example, it is assumed that the sampling frequency is 20 Hz.

(1) Filtering process using digital filter

A data array associated with the relative change over time $\Delta Q$ or the detection signal S1 which is acquired at intervals of a predetermined period is defined as X(n). Here, n is an integer. By multiplying each data piece by, for example, the following filter coefficient A(n) with n=0 in the data array X(n) as the center of time, a non-recursive type digital linear phase filter is realized.

A(0)=0.8875
A(1)=A(-1)=0
A(2)=A(-2)=-0.075
A(3)=A(-3)=0
A(4)=A(-4)=-0.0675
A(5)=A(-5)=0
A(6)=A(-6)=-0.0613
A(7)=A(-7)=0
A(8)=A(-8)=-0.06
A(9)=A(-9)-0
A(10)=A(-10)=-0.05
A(11)=A(-11)=0
A(12)=A(-12)=-0.0363
A(13)=A(-13)=0
A(14)=A(-14)--0.0338
A(15)=A(-15)=0
A(16)=A(-16)=-0.0313
A(17)=A(-17)=0
A(18)=A(-18)=-0.0288

More specifically, a delay operator of the data array X(n) is expressed by Expression (9). Here, f denotes a time frequency (unit: 1 /see). ω denotes an angular frequency and ω=2πf is established. T denotes a period in which the data array X(n) is acquired and is set to, for example, a period of 1/20 seconds to measure a change waveform to about 150 per minute (2.5 Hz).
[Math. 9]

$$e^{j\omega nT} = COS(\omega nT) + jSIN(\omega nT)$$
$$e^{-j\omega nT} = COS(\omega nT) - jSIN(\omega nT)$$
$$\cdots \ (9)$$

At this time, digital filter characteristics when the above-mentioned filter coefficient A(n) is used are described by Expression (10).
[Math. 10]

$$
\begin{aligned}
R(\omega) &= 0.8875 - 0.075\left(e^{-2j\omega T} + e^{+2j\omega T}\right) - 0.0675\left(e^{-4j\omega T} + e^{+4j\omega T}\right) - 0.0613\left(e^{-6j\omega T} + e^{+6j\omega T}\right) \\
&\quad - 0.06\left(e^{-8j\omega T} + e^{+8j\omega T}\right) - 0.05\left(e^{-10j\omega T} + e^{+10j\omega T}\right) - 0.0363\left(e^{-12j\omega T} + e^{+12j\omega T}\right) - 0.0338\left(e^{-14j\omega T} + e^{+14j\omega T}\right) \\
&\quad - 0.0313\left(e^{-16j\omega T} + e^{+16j\omega T}\right) - 0.0288\left(e^{-18j\omega T} + e^{+18j\omega T}\right) \\
&= 0.8875 - 0.075 \times 2 \times \cos(2\omega T) - 0.0675 \times 2 \times \cos(4\omega T) - 0.0613 \times 2 \times \cos(6\omega T) - 0.06 \times 2 \times \cos(8\omega T) \\
&\quad - 0.05 \times 2 \times \cos(10\omega T) - 0.0363 \times 2 \times \cos(12\omega T) - 0.0338 \times 2 \times \cos(14\omega T) - 0.0313 \times 2 \times \cos(16\omega T) \\
&\quad - 0.0288 \times 2 \times \cos(18\omega T)
\end{aligned}
$$

$$\cdots \ (10)$$

In this way, the digital filter is expressed by a product-sum operation of the data array X(n) and corresponding coefficients. The time frequency f in Expression (10) is converted to a time frequency F per minute (unit: 1 /min) to acquire Expression (11).
[Math. 11]

$$R(F) = 0.8875 - 0.075 \times 2 \times \cos(\frac{2\pi}{600}F) - 0.0675 \times 2 \times \cos(\frac{4\pi}{600}F) - 0.0613 \times 2 \times \cos(\frac{6\pi}{600}F)$$

$$- 0.06 \times 2 \times \cos(\frac{8\pi}{600}F) - 0.05 \times 2 \times \cos(\frac{10\pi}{600}F) - 0.0363 \times 2 \times \cos(\frac{12\pi}{600}F) - 0.0338 \times 2 \times \cos(\frac{14\pi}{600}F)$$

$$- 0.0313 \times 2 \times \cos(\frac{16\pi}{600}F) - 0.0288 \times 2 \times \cos(\frac{18\pi}{600}F)$$

··· （11）

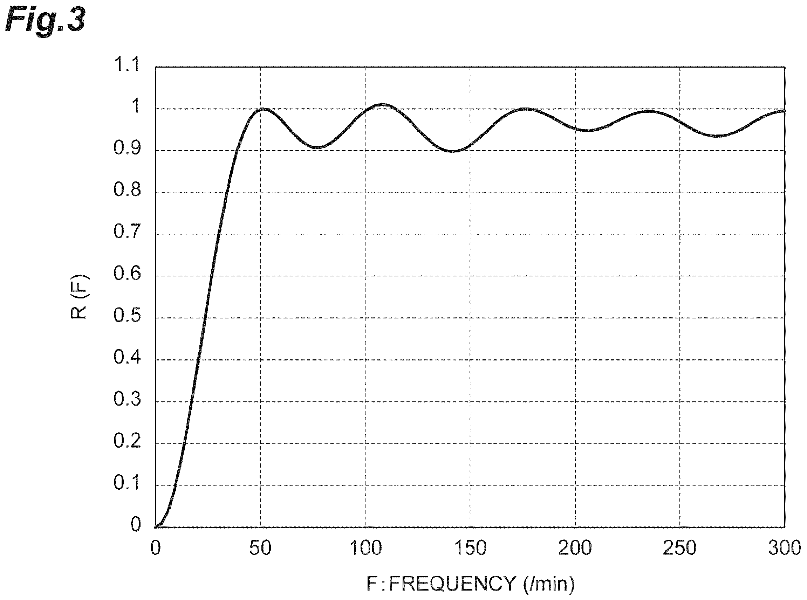

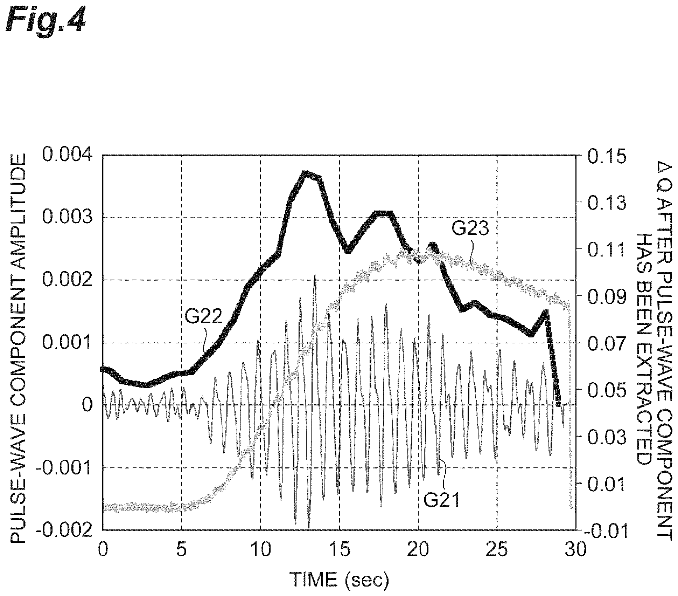

FIG. 3 illustrates R(F) in a graph and illustrates filter characteristics of a digital filter. In FIG. 3, the horizontal axis represents the heart rate per minute and the vertical axis represents the value of F(F). FIG. 4 is a graph illustrating results of removing (reducing) frequency components less than a predetermined frequency from frequency components included in the relative change over time $\Delta Q$ of the ICG concentration using the digital filter illustrated in FIG. 3 and extracting a pulse-wave component based on a heartbeat. In FIG. 4, graph G21 indicates the pulse-wave component extracted by the filtering process, graph G22 indicates the change over time of the amplitude of the pulse-wave component, and graph G23 indicates the relative change $\Delta Q$ from which the pulse-wave component is removed (that is, in the vein). The left vertical axis represents the pulse-wave component and the magnitude of the amplitude thereof (unit: mg/l·cm), the right vertical axis represents the magnitude of the relative change $\Delta Q$ (unit: mg/l·cm) from which the pulse-wave component is removed, and the horizontal axis represents time (unit: seconds). As illustrated in FIG 4, it is possible to appropriately extract the pulse-wave component due to heartbeats by the digital filter.

(2) Filtering process using smoothing operation (least-squares-error curve fitting)

With n=0 in the data array X(n) as the center of time, least-squares-error curve fitting using a high-order function (for example, a quartic function) is performed on the data array X(n) acquired in a predetermined time (for example, 3 seconds corresponding to five heartbeats) before and after. A constant term of the acquired high-order function is considered as a smoothed component (a frequency component less than a predetermined frequency) at n=0. That is, by subtracting the smoothed frequency component from the original data X(0), it is possible to remove the frequency component less than the predetermined frequency out of the frequency components included in the relative change and to separate and extract the pulse-wave component due to the heartbeats.

(3) Filtering process of arranging a maximum part or a minimum part of fluctuation in order

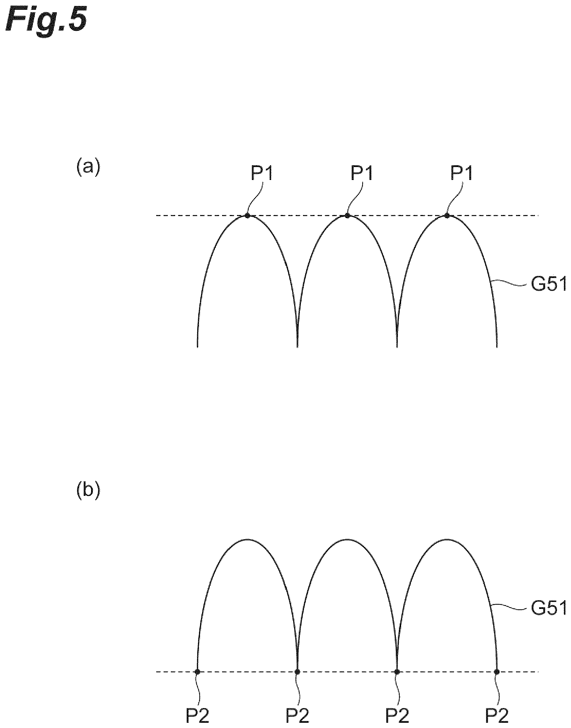

FIGS. 5(a) and 5(b) are diagrams illustrating the concept of the filtering process. In the filtering process, the frequency components which are included in the relative change $\Delta Q$ and which are less than the predetermined frequency are removed, for example, by calculating a maximum value in the change over time of the relative change $\Delta Q$ and considering the maximum value P1 of graph G51 of the change over time as a constant value as illustrated in FIG. 5(a). Alternatively, the frequency components which are included in the relative change $\Delta Q$ and which are less than the predetermined frequency are removed, for example, by calculating a minimum value in the change over time of the relative change $\Delta Q$ and considering the minimum value P2 of graph G51 of the change over time as a constant value as illustrated in FIG. 5(b). By causing the maximum value P1 and/or the minimum value P2 to approach a constant value in this way, it is possible to appropriately extract the pulse-wave component due to heartbeats.

The pulse-wave component which is extracted using the above-mentioned method is a component of the relative change $\Delta Q$ and is not an absolute value (a quantitative value). Therefore, in this embodiment, the absolute concentration of the tracer which is measured by the measurement unit 20 is used to calculate the change over time of the absolute concentration Pa of the tracer in the arteries of the head H from the pulse-wave component. That is, by measuring the absolute concentration of the tracer using the measurement unit 20 at a certain time during measurement (for example, a time at which the relative change $\Delta Q$ of the tracer concentration reaches a peak), it is possible to calculate the peak value of the change over time of the absolute concentration of the tracer. Then, by comparing the peak value of the absolute concentration of the tracer with the change over time of the amplitude of the pulse-wave component of the relative change $\Delta Q$, the absolute value of the amplitude of the pulse-wave component at each time becomes clear. Accordingly, it is possible to quantitatively ascertain the change over time of the absolute concentration Pa of the tracer in the arteries of the head H and to calculate cerebral blood flow on the basis of Expression (4).

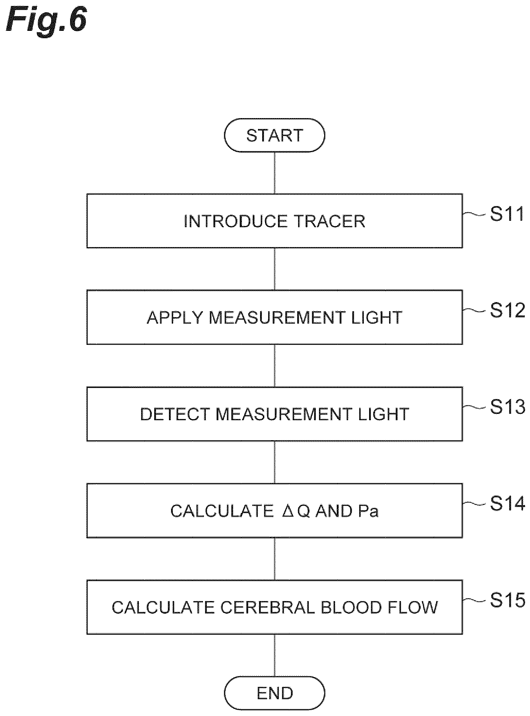

An operation method of the measurement device 1A (that is, the cerebral blood flow measurement method) according to this embodiment will be described below. FIG. 6 is a flowchart illustrating steps of the operation method of the measurement device 1A (the cerebral blood flow measurement method).

First, a tracer is introduced into a blood vessel of a sample subject (a tracer introducing step S11). Introduction of a tracer is performed by intra-arterial injection or intravenous injection to a region other than the head H of the sample subject. The types of the tracer are the same as described above. Then, the light source 3 sequentially outputs measurement light L1 of wavelengths $\lambda_1$, $\lambda_2$, and $\lambda_3$ on the basis of an instruction from the calculation unit 10. The measurement light L1 reaches the surface of the head H and is applied into the head H (a light applying step S12). The measurement light L1 introduced into the head H propagates while being scattered in the head H and being absorbed by the tracer, and some light reaches the surface of the head H. The measurement light L1 reaching the surface of the head H is detected by the light detecting element 41 (a light detecting step S13). The light detecting element 41 generates a photoelectric current corresponding to the intensity of the detected measurement light L1. The photoelectric current is converted into a voltage signal (a detection signal) by the preamplifier 42, is converted into a digital signal by an A/D conversion circuit, and is sent to the calculation unit 10 of the main body 6.

Subsequently, the calculation unit 10 calculates the change over time of the relative change $\Delta Q$ of the tracer concentration in the brain tissue and the change over time of the absolute tracer concentration Pa in the arteries of the head H on the basis of the digital signal (a calculation step S14). At this time, the calculation unit 10 extracts pulse-wave components by performing a filtering process on the digital signal or the relative change $\Delta Q$ to remove the frequency components less than the predetermined frequency. The measurement unit 20 measures the absolute tracer concentration in the arteries of the head H at least once by a dye dilution method. The calculation unit 10 calculates the change over time of the absolute tracer concentration Pa using the change over time of the amplitude of the pulse-wave component of the relative change $\Delta Q$ and the absolute tracer concentration in the arteries at a certain time point. Details of the method of calculating the relative change $\Delta Q$ and the change over time of the absolute tracer concentration Pa and the filtering process are the same as described above. Then, cerebral blood flow is calculated on the basis of a predetermined relationship (see Expression (4)) between the relative change $\Delta Q$, the change over time of the absolute tracer concentration Pa, and the cerebral blood flow (a calculation step S15).

Advantageous effects which are achieved by the measurement device 1A and the measurement method according to the above-mentioned embodiment will be described below. When a tracer is introduced into a blood vessel, a tracer concentration in a brain tissue increases gradually. A rate of increase thereof is proportional to a cerebral blood flow rate. In this embodiment, measurement light L1 including an absorption wavelength of the tracer is applied to the head H and light intensity of the measurement light L1 passing through the head H is detected. At this time, since the measurement light L1 is absorbed according to the tracer concentration in the brain tissue, a relative change $\Delta Q$ can be calculated on the basis of a predetermined relationship between the amount of absorbed light and the relative change over time $\Delta Q$ of the tracer concentration (see Expression (8)).

The relative change over time $\Delta Q$ of the tracer concentration in the brain tissue, the change over time of the tracer concentration Pa in the arteries of the head H, and the cerebral blood flow F satisfy the relationship of Expression (1). The inventor found that there is a close relationship between the change over time of the amplitude of the pulse-wave component of the relative change $\Delta Q$ and the change over time of the tracer concentration Pa in the arteries of the head H. In this embodiment, since the change over time of the tracer concentration Pa is calculated using the change over time of the amplitude of the pulse-wave component of the relative change $\Delta Q$, it is possible to calculate the cerebral blood flow F. Accordingly, it is possible to quantitatively measure cerebral blood flow using a simple device. Since the change over time of the concentration Pa in a measurement target region can be directly measured, it is possible to improve measurement accuracy. The inventor ascertained that the cerebral blood flow acquired using the method according to this embodiment has a value equivalent to the cerebral blood flow which is measured using the PET.

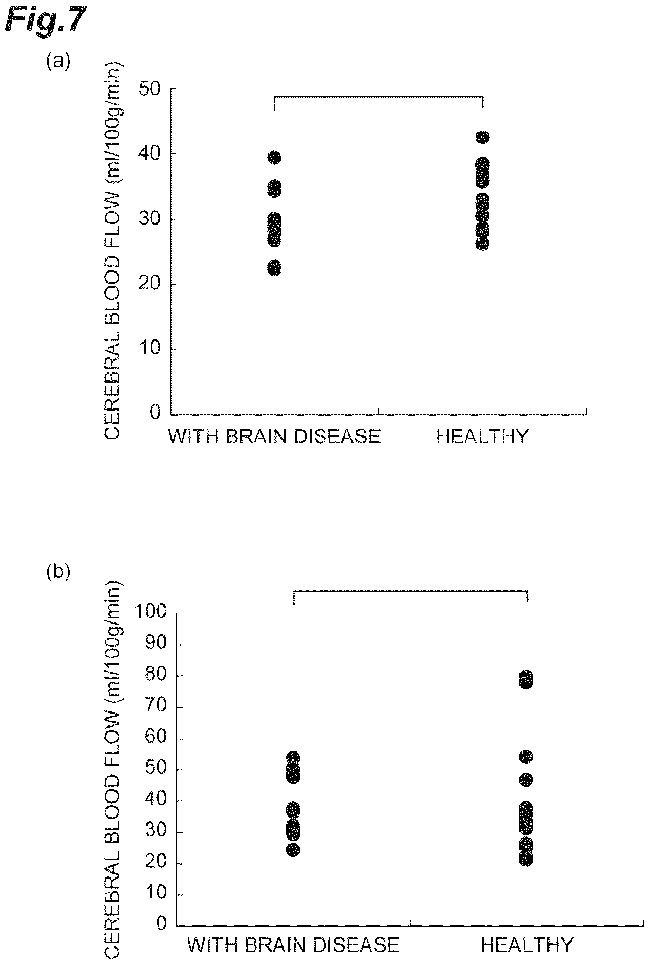

FIG. 7(a) is a graph illustrating cerebral blood flows of a plurality of sample subjects which are measured using the PET and FIG. 7(b) is a graph illustrating cerebral blood flows which are measured from the same sample subjects at the same time as measured using the PET using the measurement device 1A and the measurement method according to this embodiment. In FIGS. 7(a) and 7(b), the left plotted array indicates a result of measurement of a sample subject with a brain disease (moyamoya disease) and the right plotted array indicates a result of measurement in a normal region of the same sample subject. In the drawings, the vertical axis represents cerebral blood flow (unit: ml/100g/min). Referring to FIGS. 7(a) and 7(b), when measurement is performed using the PET, the cerebral blood flow with a brain disease is in a range of 29.1±4.4 (ml/100 g/min) and the cerebral blood flow in a healthy state is in a range of 33.3±4.6 (ml/100 g/min). When measurement is performed using the measurement device 1A and the measurement method according to this embodiment, the cerebral blood flow with a brain disease is in a range of 39.3±18.3 (ml/100 g/min) and the cerebral blood flow in a healthy state is in a range of 46.5±21.7 (ml/100 g/min). Accordingly, a difference between a diseased region and a healthy region is detected from these graphs by the measurement device 1A and the measurement method according to this embodiment and it can be seen that it is possible to accurately measure cerebral blood flow.

As in this embodiment, in the calculation step S14 (the calculation unit 10), the pulse-wave component may be extracted by performing the filtering process of removing the frequency components less than the predetermined frequency on the detection signal S1 or the relative change $\Delta Q$. Accordingly, it is possible to easily and accurately extract a pulse-wave component of the relative change $\Delta Q$ and to acquire the amplitude of the pulse-wave component. In this case, the predetermined frequency may be equal to or greater than 10 Hz and equal to or less than 100 Hz. According to the inventor's knowledge, it is possible to accurately extract a pulse-wave component by removing the frequency components less than such a frequency.

As in this embodiment, the sampling frequency of the relative change $\Delta Q$ may be greater than 10 Hz. In general, a heart rate of a person in a stabilized state ranges from about 60 per minute to 75 per minute (that is, about 1 Hz to 1.25 Hz). Accordingly, for example, by measuring the relative change $\Delta Q$ at a sampling frequency sufficiently greater (a sampling period shorter) than the heart rate in this way, it is possible to appropriately acquire the amplitude of the pulse-wave component of the relative change $\Delta Q$.

As in this embodiment, in the calculation step S14 (the calculation unit 10), a change over time of the tracer concentration Pa may be calculated on the basis of the change over time of the amplitude of the pulse-wave component and the absolute concentration of the tracer in the arteries of the head H at a certain time measured, for example, using a dye dilution method. Accordingly, it is possible to appropriately calculate the change over time of the tracer concentration Pa.

As in this embodiment, ICG may be used as the tracer. The ICG has an absorption wavelength in the near-infrared band, is used for various types of inspection in the related art, and is safe and inexpensive. Accordingly, by using the ICG as the tracer, it is possible to safely and inexpensively measure cerebral blood flow.

As in this embodiment, the absorption wavelength of the tracer may be included in the near-infrared band. Since light in the near-infrared band can easily pass through various tissues of the head H, it is possible to accurately measure a tracer concentration using a tracer of which the absorption wavelength is included in the near-infrared band.

(Modified example)

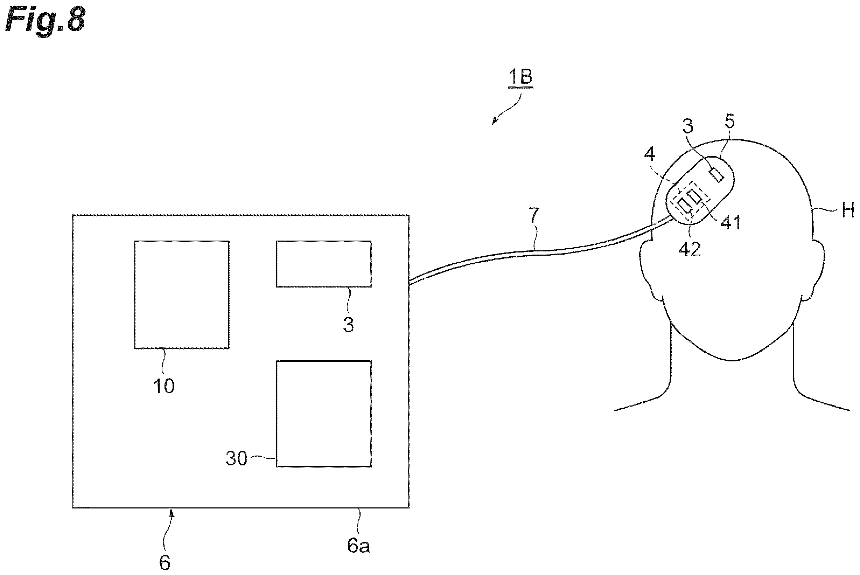

A modified example of the embodiment will be described below. FIG. 8 is a diagram schematically illustrating a configuration of a measurement device 1B according to this modified example. The measurement device 1B according to this modified example includes a measurement unit 30 instead of the measurement unit 20 in the above-mentioned embodiment. The measurement unit 30 is a unit that measures an absolute hemoglobin concentration in arterial blood in advance before introducing a tracer. The measurement unit 30 measures the absolute hemoglobin concentration in the arterial blood, for example, by analyzing blood collected from a sample subject.

In this modified example, the light source 3 applies measurement light L1 including an absorption wavelength of hemoglobin to the head H. Here, since the absorption wavelength of hemoglobin is close to the absorption wavelength of the ICG, the wavelengths exemplified above in the embodiment can be used without any change.

In this modified example, the calculation unit 10 additionally calculates a change over time of a relative hemoglobin concentration in a brain tissue on the basis of the detection signal S1. Specifically, the relative hemoglobin concentration includes the relative change over time $\Delta O_2Hb$ of an oxygenated hemoglobin concentration and a relative change over time $\Delta HHb$ of a deoxygenated hemoglobin concentration which are expressed by Expression (8). That is, the relative hemoglobin concentration can be calculated on the basis of Expression (8).

The calculation unit 10 calculates the absolute tracer concentration Pa on the basis of the absolute hemoglobin concentration measured by the measurement unit 30, the change over time of the amplitude of the pulse-wave component of the change over time of the relative hemoglobin concentration calculated by the calculation unit 10, and the change over time of the amplitude of the pulse-wave component of the relative change $\Delta Q$. Specifically, this calculation is performed as follows.

With the measurement device 1B, it is possible to measure the relative changes $\Delta O_2Hb$ and $\Delta HHb$ of the oxygenated hemoglobin concentration and the deoxygenated hemoglobin concentration using the near-infrared spectroscopy method. In arterial blood, an amount of deoxygenated hemoglobin is relatively small and the relative change $\Delta O_2Hb$ of the oxygenated hemoglobin is mainly measured. When this measurement is performed at a high speed (for example, 20 Hz), the change over time of the relative change $\Delta O_2Hb$ includes a pulse-wave component due to heartbeats. This pulse-wave component can be considered as a change over time of an arterial blood flow rate. The pulse-wave component can be separated and extracted from the relative change $\Delta O_2Hb$ by performing the same filtering process as on the relative change $\Delta Q$ of the tracer concentration on the relative change $\Delta O_2Hb$.

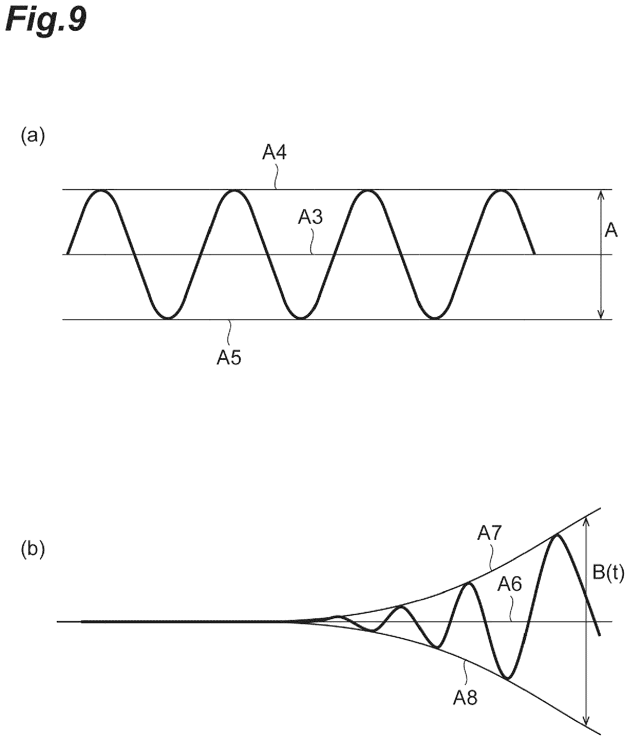

FIG. 9(a) is a graph conceptually illustrating a change over time of a pulse-wave component which is extracted from the relative change $\Delta O_2Hb$. In the drawing, a straight line A3 indicates the center of the amplitude of the pulse-wave component, a straight line A4 indicates an upper limit of the amplitude of the pulse-wave component, and a straight line A5 indicates a lower limit of the amplitude of the pulse-wave component. In general, when the position of the probe 5 does not change, the amplitude A of the change over time of the pulse-wave component of the relative change $\Delta O_2Hb$ is constant regardless of time as illustrated in the drawing.

On the other hand, when a tracer is injected into a blood vessel, the relative change over time $\Delta Q$ of the tracer concentration in a brain tissue increases while including a pulse-wave component as described above in the embodiment. As described above in the embodiment, the pulse-wave component is also separated and extracted by the filtering process. FIG. 9(b) is a graph conceptually illustrating a change over time of the pulse-wave component extracted from the relative change ΔQ. In the drawing, a straight line A6 indicates the center of the amplitude of the pulse-wave component, a curve A7 indicates an upper limit of the amplitude of the pulse-wave component, and a curve A8 indicates a lower limit of the amplitude of the pulse-wave component. As illustrated in the drawing, the amplitude B(t) of the change over time of the pulse-wave component of the relative change $\Delta O_2 Hb$ increases with an increase in the tracer concentration in arteries.

Here, a ratio of the amplitude A to the amplitude B(t) at time t matches a ratio of the hemoglobin concentration in arterial blood to the absolute tracer concentration at time t. That is, by measuring the amplitude A and the amplitude B(t), it is possible to measure the absolute tracer concentration Pa(t) at time t. The hemoglobin concentration in arterial blood is measured by the measurement unit 30.

Specifically, the following expression is established.

$$A:B(t) = Hb/(Hb\_M):Pa(t)/TR\_M$$

Here, Hb (g/dl) denotes a hemoglobin concentration in arterial blood which is measured by the measurement unit 30, and Hb_M denotes a molecular weight of hemoglobin (64500), Pa(t) (mg/dl) denotes an absolute tracer concentration at time t, and TR_M denotes a molecular weight of a tracer (775 in case of ICG). Accordingly, the absolute tracer concentration Pa(t) is calculated by the following expression.

$$Pa(t) = B(t) \cdot (Hb \cdot TR\_M)/(A \cdot Hb\_M)$$

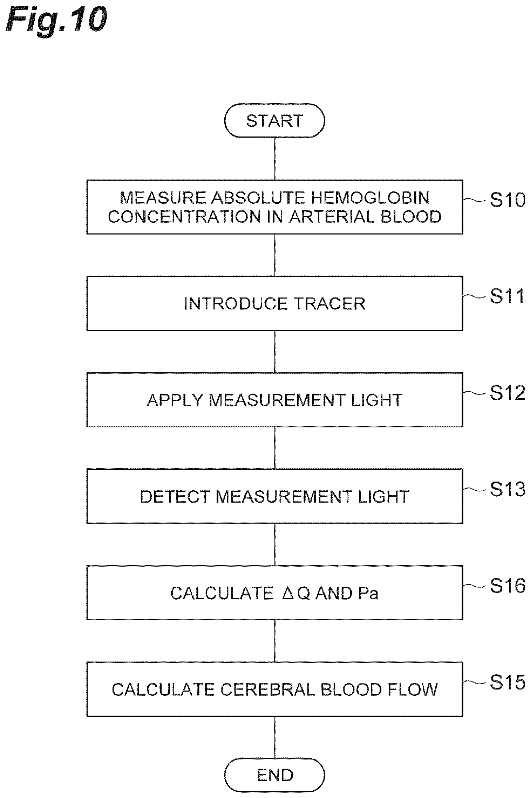

FIG. 10 is a flowchart illustrating an operation method of the measurement device 1B (a cerebral blood flow measurement method) according to this modified example. As illustrated in FIG. 10, in this modified example, first, the measurement unit 30 measures the absolute hemoglobin concentration in arterial blood in advance before introducing the tracer (a hemoglobin measuring step S10). Then, similarly to the embodiment, the tracer introducing step S11, the light applying step S12, and the light detecting step S13 are performed. Subsequently, in the calculation step S16, the relative change over time $\Delta O_2 Hb$ of the oxygenated hemoglobin concentration in addition to the relative change over time ΔQ of the tracer concentration is calculated. Then, the filtering process is performed on the relative changes ΔQ and $\Delta O_2 Hb$ and pulse-wave components are extracted. The absolute tracer concentration Pa(t) is calculated on the basis of the amplitude A and B(t) of the pulse-wave components and the absolute hemoglobin concentration in arterial blood measured by the measurement unit 30. Thereafter, similarly to the embodiment, the calculation step S15 is performed to calculate cerebral blood flow.

According to the above-mentioned modified example, similarly to the embodiment, it is possible to quantitatively measure cerebral blood flow using a simple device. According to the modified example, the measurement unit 20 in the embodiment (for example, the DDG analyzer) is not necessary and thus it is possible to contribute to simplification of a device configuration and a decrease in size.

The cerebral blood flow measurement method and the cerebral blood flow measurement device according to an aspect of the invention are not limited to the above-mentioned embodiment and can be modified in various forms. For example, indocyanine green is exemplified as a tracer in the embodiment, but the tracer is not limited thereto as long as it can absorb light of a specific wavelength. For example, various tracers such as methylene blue, patent blue, and indigo carmine can be used. Various high-pass filters are exemplified as an example of a filter in the embodiment, but a pulse-wave component can be appropriately extracted from the relative change ΔQ using a band-pass filter.

**Industrial Applicability**

It is possible to quantitatively measure cerebral blood flow using a simple device.

**Reference Signs List**

1A, 1B Measurement device
3 Light source
4 Light detector
5 Probe
6 Main body

6a Casing
10 Calculation unit
20 Measurement unit
21 Probe
30 Measurement unit
41 Light detecting element
42 Preamplifier
H Head
L1 Measurement light
S1 Detection signal
S10 Hemoglobin measuring step
S11 Tracer introducing step
S12 Light applying step
S13 Light detecting step
S14 to S16 Calculation step

**Claims**

1. A cerebral blood flow measurement method of quantitatively measuring cerebral blood flow, the measurement method comprising:

   a tracer introducing step of introducing a tracer into a blood vessel;
   a light applying step of applying measurement light including an absorption wavelength of the tracer to a head;
   a light detecting step of detecting the measurement light propagating in the head and generating a detection signal based on light intensity of the measurement light; and
   a calculation step of calculating cerebral blood flow on the basis of a relative change over time $\Delta Q$ of a concentration of the tracer in a brain tissue which is acquired on the basis of the detection signal and a predetermined relationship between a change over time of a concentration Pa of the tracer in an artery of the head and cerebral blood flow,
   wherein the calculation step includes calculating the change over time of the concentration Pa using a change over time of an amplitude of a pulse-wave component of the relative change $\Delta Q$.

2. The cerebral blood flow measurement method according to claim 1, wherein the calculation step includes extracting the pulse-wave component by performing a filtering process of removing a frequency component lower than a predetermined frequency on the detection signal.

3. The cerebral blood flow measurement method according to claim 1, wherein the calculation step includes extracting the pulse-wave component by performing a filtering process of removing a frequency component lower than a predetermined frequency on the relative change $\Delta Q$.

4. The cerebral blood flow measurement method according to claim 2 or 3, wherein the predetermined frequency is equal to or greater than 10 Hz and equal to or less than 100 Hz.

5. The cerebral blood flow measurement method according to any one of claims 1 to 4, wherein a sampling frequency of the relative change $\Delta Q$ is greater than 10 Hz.

6. The cerebral blood flow measurement method according to any one of claims 1 to 5, wherein the calculation step includes calculating a change over time of the concentration Pa on the basis of the change over time of the amplitude of the pulse-wave component and an absolute concentration of the tracer in an artery of the head at a certain time.

7. The cerebral blood flow measurement method according to claim 6, wherein the absolute concentration of the tracer in an artery of the head is measured using a dye dilution method.

8. The cerebral blood flow measurement method according to any one of claims 1 to 5, further comprising a hemoglobin measuring step of measuring an absolute hemoglobin concentration in an artery in advance before the tracer introducing step,
   wherein the light applying step includes applying the measurement light including an absorption wavelength of hemoglobin to the head, and

wherein the calculation step includes calculating a change over time of a relative hemoglobin concentration in a brain tissue on the basis of the detection signal and calculating the change over time of the concentration Pa on the basis of the absolute hemoglobin concentration, the change over time of the amplitude of the pulse-wave component of the change over time of the relative hemoglobin concentration, and the change over time of the amplitude of the pulse-wave component of the relative change $\Delta Q$.

9. The cerebral blood flow measurement method according to any one of claims 1 to 8, wherein indocyanine green is used as the tracer.

10. The cerebral blood flow measurement method according to any one of claims 1 to 9, wherein the absorption wavelength of the tracer is included in a near-infrared band.

11. A cerebral blood flow measurement device that quantitatively measures cerebral blood flow, the measurement device comprising:

a light applying unit configured to apply measurement light including an absorption wavelength of a tracer which is introduced into a blood vessel to a head;

a light detecting unit configured to detect the measurement light propagating in the head and generates a detection signal based on light intensity of the measurement light; and

a calculation unit configured to calculate cerebral blood flow on the basis of a relative change over time $\Delta Q$ of a concentration of the tracer in a brain tissue which is acquired on the basis of the detection signal and a predetermined relationship between a change over time of a concentration Pa of the tracer in an artery of the head and cerebral blood flow,

wherein the calculation unit calculates the change over time of the concentration Pa using a change over time of an amplitude of a pulse-wave component of the relative change $\Delta Q$.

12. The cerebral blood flow measurement device according to claim 11, wherein the calculation unit extracts the pulse-wave component by performing a filtering process of removing a frequency component lower than a predetermined frequency on the detection signal.

13. The cerebral blood flow measurement device according to claim 11, wherein the calculation unit extracts the pulse-wave component by performing a filtering process of removing a frequency component lower than a predetermined frequency on the relative change $\Delta Q$.

14. The cerebral blood flow measurement device according to claim 12 or 13, wherein the predetermined frequency is equal to or greater than 10 Hz and equal to or less than 100 Hz.

15. The cerebral blood flow measurement device according to any one of claims 11 to 14, wherein a sampling frequency of the relative change $\Delta Q$ is greater than 10 Hz.

16. The cerebral blood flow measurement device according to any one of claims 11 to 15, wherein the calculation unit calculates a change over time of the concentration Pa on the basis of the change over time of the amplitude of the pulse-wave component and an absolute concentration of the tracer in an artery of the head at a certain time.

17. The cerebral blood flow measurement device according to claim 16, further comprising a measurement unit configured to measure the absolute concentration of the tracer in an artery of the head using a dye dilution method.

18. The cerebral blood flow measurement device according to any one of claims 11 to 15, further comprising a measurement unit configured to measure an absolute hemoglobin concentration in an artery in advance before introducing the tracer,

wherein the light applying unit applies the measurement light including an absorption wavelength of hemoglobin to the head, and

wherein the calculation unit calculates a change over time of a relative hemoglobin concentration in a brain tissue on the basis of the detection signal and calculates the change over time of the concentration Pa on the basis of the absolute hemoglobin concentration, the change over time of the amplitude of the pulse-wave component of the change over time of the relative hemoglobin concentration, and the change over time of the amplitude of the pulse-wave component of the relative change $\Delta Q$.

19. The cerebral blood flow measurement device according to any one of claims 11 to 18, wherein indocyanine green is used as the tracer.

20. The cerebral blood flow measurement device according to any one of claims 11 to 19, wherein the absorption wavelength of the tracer is included in a near-infrared band.

**Fig.1**

**Fig.2**

Fig.3

EP 3 701 861 A1

# Fig.4

**Fig.5**

(a)

(b)

## Fig.6

START

INTRODUCE TRACER — S11

APPLY MEASUREMENT LIGHT — S12

DETECT MEASUREMENT LIGHT — S13

CALCULATE $\triangle Q$ AND Pa — S14

CALCULATE CEREBRAL BLOOD FLOW — S15

END

# Fig.7

(a)

(b)

**Fig.8**

# Fig.9

(a)

(b)

*Fig.10*

```
          ( START )
              |
  ┌───────────────────────────────┐
  │ MEASURE ABSOLUTE HEMOGLOBIN   │~ S10
  │ CONCENTRATION IN ARTERIAL BLOOD│
  └───────────────────────────────┘
              |
  ┌───────────────────────────────┐
  │       INTRODUCE TRACER        │~ S11
  └───────────────────────────────┘
              |
  ┌───────────────────────────────┐
  │    APPLY MEASUREMENT LIGHT    │~ S12
  └───────────────────────────────┘
              |
  ┌───────────────────────────────┐
  │    DETECT MEASUREMENT LIGHT   │~ S13
  └───────────────────────────────┘
              |
  ┌───────────────────────────────┐
  │    CALCULATE △Q AND Pa        │~ S16
  └───────────────────────────────┘
              |
  ┌───────────────────────────────┐
  │  CALCULATE CEREBRAL BLOOD FLOW│~ S15
  └───────────────────────────────┘
              |
          ( END )
```

**INTERNATIONAL SEARCH REPORT**

| International application No. |
|---|
| PCT/JP2018/039524 |

| A. CLASSIFICATION OF SUBJECT MATTER |
|---|
| Int.Cl. A61B5/0275(2006.01)i, A61B10/00(2006.01)i |
| According to International Patent Classification (IPC) or to both national classification and IPC |

| B. FIELDS SEARCHED |
|---|
| Minimum documentation searched (classification system followed by classification symbols) |
| Int.Cl. A61B5/0275, A61B10/00 |

| Documentation searched other than minimum documentation to the extent that such documents are included in the fields searched |
|---|
| Published examined utility model applications of Japan    1922–1996 |
| Published unexamined utility model applications of Japan    1971–2019 |
| Registered utility model specifications of Japan    1996–2019 |
| Published registered utility model applications of Japan    1994–2019 |

| Electronic data base consulted during the international search (name of data base and, where practicable, search terms used) |
|---|
| |

C. DOCUMENTS CONSIDERED TO BE RELEVANT

| Category* | Citation of document, with indication, where appropriate, of the relevant passages | Relevant to claim No. |
|---|---|---|
| A | JP 2003-144401 A (SHIMADZU CORPORATION) 20 May 2003, paragraphs [0018]-[0043], fig. 1-5 (Family: none) | 1-20 |
| A | JP 2017-113637 A (ST. MARIANNA UNIVERSITY SCHOOL OF MEDICINE) 29 June 2017, entire text, all drawings (Family: none) | 1-20 |
| A | JP 6-277202 A (HAMAMATSU PHOTONICS K.K.) 04 October 1994, entire text, all drawings & US 5564418 A, entire text, all drawings & EP 0616791 A2 | 1-20 |
| A | KATO, Shinya et al., "Cerebral Blood Flow Measurement by Near-Infrared Spectroscopy During Carotid Endarterectomy", JOURNAL OF NEUROSURGICAL ANESTHESIOLOGY 31 October 2016, vol. 28, no. 4, pp. 291-295 | 1-20 |
| A | JP 10-509629 A (HOEFT, Andreas) 22 September 1998, entire text, all drawings & US 5865757 A, entire text, all drawings | 1-20 |

☒ Further documents are listed in the continuation of Box C.    ☐ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 07 January 2019 (07.01.2019) | 15 January 2019 (15.01.2019) |

| Name and mailing address of the ISA/ | Authorized officer |
|---|---|
| Japan Patent Office<br>3-4-3, Kasumigaseki, Chiyoda-ku,<br>Tokyo 100-8915, Japan | |
| | Telephone No. |

Form PCT/ISA/210 (second sheet) (January 2015)

**INTERNATIONAL SEARCH REPORT**

| International application No. |
| --- |
| PCT/JP2018/039524 |

C (Continuation).    DOCUMENTS CONSIDERED TO BE RELEVANT

| Category* | Citation of document, with indication, where appropriate, of the relevant passages | Relevant to claim No. |
| --- | --- | --- |
| A | JP 2002-509453 A (PULSION MEDICAL SYSTEMS AG) 26 March 2002, entire text, all drawings & US 6223069 B1, entire text, all drawings | 1-20 |

Form PCT/ISA/210 (continuation of second sheet) (January 2015)

27

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **H. W. SCHYTZ et al.** Changes in cerebral blood flow after acetazolamide: an experimental study comparing near-infrared spectroscopy and SPECT. *Eur J Neurol. Author manuscript; available in PMC,* 25 November 2009
- **BENDICHT P. WAGNER et al.** Reproducibility of the blood flow index as noninvasive, bedside estimation of cerebral blood flow. *Intensive Care Med29,* 2003, 196-200
- **CHRISTOPH TERBORG et al.** Noninvasive Assessment of Cerebral Perfusion and Oxygenation in Acute Ischemic Stroke by Near-Infrared Spectroscopy. *European Neurology,* 2009, vol. 62, 338-343
- **FELIX GORA et al.** Noninvasive Measurement of Cerebral Blood Flow in Adults Using Near-Infrared Spectroscopy and Indocyanine Green: A Pilot Study. *Journal of Neurosurgical Anesthesiology,* vol. 14 (3), 218-222
- **KUEBLER WM ; SCKELL A ; HABLER O. et al.** Noninvasive measurement of regional cerebral blood flow by near-infrared spectroscopy and indocyanine green. *J Cereb Blood Metab,* 1998, vol. 18, 445-456
- **KATO S ; YOSHITANI K ; OHNISHI Y.** Cerebral Blood Flow Measurement by Near-Infrared Spectroscopy During Carotid Endarterectomy. *Journal of Neurosurgical Anesthesiology,* 2016, vol. 28 (4), 291-295
- **TAKEHIKO IIJIMA et al.** Cardiac Output And Circulating Blood Volume Analysis By Pulse Dye-Densitometry. *journal of Clinical Monitoring,* 1997, vol. 13 (2), 81-89
- **TAKASUKE IMAI et al.** Measurement of Cardiac Output by Pulse Dye Densitometry Using Indocyanine Green. *Anesthesiology,* 1997, vol. 87 (4
- **MASAKI HARUNA et al.** Blood Volume Measurement at the Bedside Using ICG Pulse Spectrophotometry. *Anesthesiology,* 1998, vol. 89 (6
- **TAKEHIKO IIJIMA et al.** Circulating Blood Volume Measured by Pulse Dye-Densitometry. *Anesthesiology,* 1998, vol. 89 (6
- **TAKASUKE IMAI et al.** Measurement of Blood Concentration of Indogyanine Green by Pulse Dye Densitometry Comparison with the Conventional Spectrophotometric Method. *Journal of Clinical Monitoring and Computing,* 1998, vol. 14, 477-484